(19) Europäisches Patentamt
European Patent Office
Office européen des brevets

(11) EP 0 567 462 B1

# (12) FASCICULE DE BREVET EUROPEEN

(45) Date de publication et mention de la délivrance du brevet: **27.05.1998 Bulletin 1998/22**

(21) Numéro de dépôt: **92900221.0**

(22) Date de dépôt: **13.11.1991**

(51) Int Cl.[6]: **G01N 33/543** // G01N33/68, G01N33/574

(86) Numéro de dépôt international: **PCT/FR91/00891**

(87) Numéro de publication internationale: **WO 93/10453 (27.05.1993 Gazette 1993/13)**

## (54) Dispositif et procédé de détection immunologique

Immunologisches Nachweisverfahren und Vorrichtung dafür

Immunological detection method and device

(84) Etats contractants désignés: **AT BE CH DE DK ES FR GB GR IT LI LU NL SE**

(43) Date de publication de la demande: **03.11.1993 Bulletin 1993/44**

(73) Titulaires:
- **DIAGNOSTICA STAGO (société anonyme) F-92602 Asnières (FR)**
- **ALFA BIOTECH SpA 00040 Pomezia (Roma) (IT)**

(72) Inventeur: **Toledano, Jacques F-75020 Paris (FR)**

(74) Mandataire: **Clisci, Serge et al S.A. FEDIT-LORIOT & AUTRES CONSEILS EN PROPRIETE INDUSTRIELLE 38, Avenue Hoche 75008 Paris (FR)**

(56) Documents cités:

| | |
|---|---|
| EP-A- 0 274 198 | EP-A- 0 303 110 |
| EP-A- 0 327 843 | EP-A- 0 328 106 |
| WO-A-90/03844 | FR-A- 2 514 511 |
| GB-A- 2 034 466 | US-A- 4 459 358 |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 567 462 B1

Printed by Jouve, 75001 PARIS (FR)

## Description

La présente invention est relative à un dispositif et un procédé pour la détermination qualitative et quantitative rapide d'un ligand dans un fluide. L'invention s'applique plus particulièrement, mais non limitativement, à la détection de la présence d'antigènes ou d'anticorps dans un fluide biologique, à la détection de substances toxiques, de virus ou de bactéries dans un fluide quelconque (eaux, produits fluides des industries agro-alimentaires, effluents industriels, fluides biologiques, etc..), au dosage chimique de substances (telles qu'hormones, vitamines, enzymzes, médicaments, etc..), présentes dans un fluide (notamment biologique).

Les méthodes de détermination de la présence ou de la concentration de substances antigèniques dans des fluides biologiques, par voie immunologique, sont à présent bien connues ; elles sont basées sur la formation d'un complexe entre la substance antigènique à déterminer et un ou plusieurs anticorps, l'un des composants du complexe pouvant être marqué par un élément radioactif (tel que l'Iode 125, par exemple), pour permettre sa détection et/ou son analyse quantitative après séparation de l'antigène ou de l'anticorps marqué complexé, de l'antigène ou de l'anticorps marqué non complexé.

Dans les méthodes de détermination immunologique par compétition, la substance antigènique contenue dans l'échantillon de liquide à analyser, entre en compétition avec une quantité connue d'antigène marqué pour une quantité limitée de sites de fixation de l'anticorps : la quantité d'antigène marqué liée à l'anticorps est inversement proportionnelle à la quantité d'antigène contenue dans l'échantillon.

Les tests immunométriques utilisent un antigène marqué : la quantité d'anticorps marqué associée au complexe est proportionnelle à la quantité de substance antigènique contenue dans l'échantillon.

Des tests immunométriques particulièrement adaptés à la détection d'antigènes polyvalents, c'est à dire de substances antigèniques capables de se complexer avec au moins deux anticorps en même temps, consistent à utiliser une quantité d'anticorps non marqué lié à un support solide insoluble dans le liquide à analyser, et une quantité d'anticorps soluble portant un indicateur, tel qu'un isotope radioactif, qui permet la détection ou la détermination quantitative du complexe ternaire formé entre l'anticorps en phase solide, l'antigène et l'anticorps marqué. Ces méthodes consistent à mettre tout d'abord en contact l'anticorps lié à la phase solide avec l'échantillon à analyser, pour extraire l'antigène de l'échantillon,par formation d'un complexe binaire entre l'anticorps en phase solide et l'antigène. Après une période d'incubation, le support solide est lavé pour en éliminer le résidu d'échantillon liquide, y compris l'antigène éventuel n'ayant pas réagi, puis mis en contat avec une solution contenant une quantité connue d'anticorps marqué. Après une seconde période d'incubation pour permettre à l'anticorps marqué de se complexer avec l'antigène lié au support solide par l'intermédiaire de l'anticorps non marqué, le support solide est lavé une seconde fois pour enlever l'anticorps marqué n'ayant pas réagi ; puis la présence d'anticorps marqué sur le support solide lavé est détectée (par exemple par mesure du rayonnement émis, si l'indicateur est un rayonnement radioactif) et éventuellement soumise à un processus de détermination quantitative.

Ces méthodes sont connues sous le nom de méthodes "sandwich" ou "bi-site", du fait que l'antigène porte deux anticorps liés à sa surface en deux sites différents. Elles sont décrites par WIDE dans "Radioimmunoassay Methods" (Kirkham et Hunter,E et S. Livingstone,Ed. Edimbourg,1970,p 199-206).

Des applications spécifiques (test de détection de l'antigène de l'hépatite) sont décrites dans le Brevet des Etats-Unis d'Amérique N° 3 867 517 ; des variantes de ces méthodes ("simultanées" ou "inverses") sont décrites par JEONG et al.dans "Comparison of RIA with a single incubation two-site immunoradiometric assay (IRMA) as applied to the détermination of human thyroid stimulating hormone (HTSH)", Bio-Rad Laboratories, 1979 ; dans le Brevet des Etats-Unis d'Amérique N° 4 174 384 (marquage de deux anticorps par un chromophore fluorescent (fluoresceïne) et par un chromophore qui absorbe la lumière émise par la fluoresceïne) ; dans le Brevet des Etats-Unis d'Amérique N° 4 098 876. Ces méthodes utilisaient initialement des anticorps polyclonaux,dont la sensibilité est insuffisante en raison de l'existence d'une réactivité croisée avec d'autres antigènes. Le Brevet français Hybritech N° 81 15030 publié sous le N° 2 487 983, a proposé de remplacer dans ces tests immunométriques, les anticorps polyclonaux par des anticorps monoclonaux, pour augmenter de façon importante la sensibilité de ces méthodes.

Le Brevet français LIOTTA N° 82 16973, publié sous le N° 2 514 511, décrit un dispositif et un procédé pour déterminer la présence d'antigènes dans des fluides biologiques, par la méthode ELISA, tout en éliminant les opérations de lavage et de dilution qu'elle nécessite et en réduisant la durée d'incubation. Le dispositif proposé comprend une matrice formée d'un ruban en papier de nitrocellulose ou de diazobenzyloxyméthyle (DBM), en gel poreux tel le polyacrylamide, l'agarose ou le collagène, dans les pores duquel l'antigène peut être piégé ou bien il peut être réticulé avec le gel par l'intermédiaire des groupes amines du ligand et des groupes carboxyles portés par la matrice, ou bien d'un ruban de cellulose ou de fibres de matière plastique à l'intérieur duquel sont piégées des particules ou des billes qui contiennent le ligand.Conformément à ce brevet LIOTTA, le dispositif comprend :

- une première zone qui contient des antigènes et des anticorps liés à une enzyme,ces anticorps étant situés dans la dite première zone de telle manière qu'ils soient éliminés de cette première zone quand ils ont réagi avec des antigènes non liés contenus dans l'échantillon à tester, traversant cette première zone,mais qu'ils n'en soient pas éliminés en l'absence de tels antigènes,

- et une seconde zone contenant une substance capable de réagir avec les dits anticorps liés à une enzyme, pour produire une réaction colorée révélant la présence des dits anticorps.

De façon spécifique, le dispositif de dosage immunologique décrit par le brevet Liotta, comprend un stratifié à trois couches de matrice poreuse dont la première est imprégnée d'un anticorps spécifique lié à une enzyme, la seconde couche poreuse contient un antigène de référence immobilisé (lié), et la troisième couche contient un substrat produisant une couleur, qui réagit avec l'enzyme liée à l'anticorps. L'antigène libre à doser présent dans l'échantillon à analyser, mis en contact avec la première couche, diffuse dans la seconde, puis dans la troisième couche. L'antigène libre présent dans l'échantillon entre en compétition avec l'antigène de référence lié, immobilisé dans la seconde couche, pour se combiner à l'anticorps lié à une enzyme. Si l'anticorps lié à une enzyme se combine à l'antigène libre, il diffuse librement vers la troisième couche et produit une réaction colorée. Par contre, si l'échantillon à analyser ne contient pas d'antigène,tous les anticorps liés à une enzyme possèdent des sites de liaison libres et se combinent avec l'antigène immobilisé présent dans la seconde couche ; l'anticorps lié à une enzyme, qui se combine à l'antigène immobilisé dans la seconde couche, ne diffuse pas dans la troisième couche et aucune réaction colorée ne se produit. Le brevet français LIOTTA 87 08007, publié sous le N° 2 599 845 décrit une variante de ce dispositif, qui ne comprend que deux couches, dont la couche supérieure porte l'antigène avec les deux anticorps réactifs et dont la couche inférieure contient un substrat chromogène, les deux zones (zone de piégeage et zone de substrat) pouvant également être juxtaposées.

Le Brevet américain HYBRITECH N° 4 632 901 (et la demande internationale PCT correspondante N° 85/05451) décrit un procédé et un dispositif de réalisations d'immunoassays qui ne nécessitent pas le recours à des étapes d'incubation de longue durée associées à plusieurs lavages,et permettent par leur simplicité, la mise en oeuvre par un médecin, à sa consultation, et même par le patient chez lui. Ce dispositif comprend un premier élément poreux constitué par une membrane ou un filtre auquel est lié un anticorps, monoclonal ou polyclonal, qui reconnait l'antigène que l'on cherche à identifier, et un deuxième élément, qui est absorbant et comporte des passages capillaires perpendiculaires à ses surfaces supérieure et inférieure ; ce second élément est en communication capillaire avec la membrane, ou analogue, poreuse, qui forme le premier élément du dispositif, et présente une dimension de pores telle qu'il induise la traversée de liquide à travers le premier élément sans application de moyens externes. La membrane filtrante peut être du Nylon portant des groupes NH2 auxquels les anticorps sont liés par covalence, par couplage à l'aide de glutaraldéhyde et l'élément absorbant peut être en matière filtrante fibreuse telle que fibres d'acétate de cellulose,de polyester, de polyoléfine,etc.. Ces deux éléments peuvent être séparés l'un de l'autre par un autre élément poreux (en polyéthylène par exemple) qui ne fixe pas d'anticorps de façon non-spécifique et empêche que de l'anticorps marqué ne se fixe de façon non-spécifique sur la surface supérieure de l'élément absorbant. De même la demande de Brevet Européen ABBOTT Laboratories N° 186 100 décrit un dispositif qui vise à déterminer la présence ou la quantité d'une substance dans un échantillon à tester, et qui comprend une matrice fibreuse poreuse non-absorbante, telle que de verre, imprégnée d'un polymère hydrophobe qui forme un revêtement de surface sur au moins une partie de la matrice et auquel est fixé un réactif, par exemple un anticorps ou un antigène, capable de réagir avec la substance recherchée dans l'échantillon, ce dispositif pouvant être associé à une couche de matière absorbante sous-jacente et/ou à une couche de matière fibreuse telle que fibres de verre ou papier-filtre en cellulose, qui recouvre la matrice fibreuse et peut jouer le rôle de pré-filtre.

La demande de Brevet européen MUREX Corp. N° 206 561 fait également connaître un dispositif de diagnostic qui comprend un élément filtrant comportant au moins une zone de réaction associée à au moins une zone périphérique, un élément absorbant associé uniquement à la zone périphérique du premier élément et un élément de maintien du filtre en position appropriée.La "réaction" qui se produit dans la zone de réaction peut aussi bien être une séparation par filtration qu'un couplage immunologique et c'est dans la même zone, qu'apparaissent les signaux de lecture de la réaction. L'échantillon liquide parvient sur le filtre par un entonnoir dont l'orifice de décharge est calculé de telle manière que son diamètre soit suffisant, en combinaison avec la pression du liquide dans l'entonnoir, pour que le liquide se décharge sur la surface supérieure du filtre mais ne traverse pas ce dernier, la pression hydrostatique étant, par la suite, ajustée de telle manière que le liquide pénètre dans le filtre par gravité et est diffusé à travers ce dernier par action capillaire, sans le traverser perpendiculairement de part en part.

Le Brevet Unilever PLC No EP-A-274198 est une colonne de type chromatographique à travers laquelle s'écoule le fluide de l'échantillon.Elle est constituée de support immobilisant des anticorps anti-enzymes associés à l'antigène identique à celui recherché de façon à piéger un anticorps spécifique de cet antigène.Ce dernier anticorps est en fait un quadrome : un site Fab reconnaît l'antigène tandis que le site Fab restant reconnaît une enzyme. C'est en fait un moyen de marquage original mais le procédé est en fait identique aux brevets cités plus haut : un antigène immobilisé dans la colonne piège l'anticorps marqué qui n'a pas rencontré d'antigène dans l'échantillon.

Le Brevet KONICA Corp. No EP-A-328106 est également une superposition de trois compartiments qui ont la même fonction que dans le brevet objet de la présente invention mais il s'agit encore ici d'un piégeage des anticorps marqués libres par un antigène correspondant immobilisé dans le compartiment médian.

Tous ces dispositifs ont en commun un élément filtrant,à travers lequel s'écoule l'échantillon liquide à tester.La filtration est rendue sélective par la présence à ce niveau, immobilisé, d'un ligand identique à celui recherché.

Mattiasson,Danielson,Hermansson et Mosbach (FEBS Letters. 85 : p 203,1978) ont mis en évidence les changements qui interviennent dans les structures des protéines quand ces dernières ne se trouvent plus dans leurs conditions physiologiques, et proposent toute une série de solutions intéressantes.

A l'inverse, la littérature est abondante sur les hautes qualités de résistance, de conservation et de stabilité des immunoglobulines. C'est la raison pour laquelle le procédé objet de la présente invention se propose d'utiliser des immunoglobulines convenablement préparées et choisies pour stopper un anticorps marqué quand il n'est pas saturé en antigène,et le laisser descendre vers le substrat quand il est saturé en antigène. De même, elles stoppent un antigène marqué qui n'a pas rencontré d'anticorps dans l'échantillon, et laissent passer les complexes antigènes marqués + anticorps de l'échantillon. Ces immunoglobulines fixées sur l'élément filtrant sélectif, remplissent cette mission par action soit sur l'enzyme qui marque l'anticorps (ou l'antigène) déposé sur la première couche, soit sur l'anticorps marqué lui même (en se fixant sur ses sites Fab restés libres, par exemple), soit sur les deux : les immunoglobulines fixent l'enzyme de marquage (en l'absence de complexe antigène + anticorps) et l'anticorps (ou l'antigène) supportant ce marquage (et restés insaturés par le ligand correspondant).

Dans ces conditions, à la différence de tous les procédés cités plus haut, l'élément filtrant sélectif acquiert un caractère universel : qui ne dépend plus spécifiquement du ligand recherché. Exemple : en déposant sur la couche supérieure du dispositif un anticorps marqué du type IgG1 de souris, spécifique de la FSH humaine (Follicle stimulating hormone), et marquée d'une certaine façon par la peroxydase : l'élément filtrant sélectif comprend, immobilisé, une immunoglobuline antiperoxydase convenablement sélectionnée et preparée : si l'échantillon ne contient pas de FSH, l'IgG1 de souris, marquée à la peroxydase, non saturée en antigène FSH, va être stoppée dans sa migration (vers la zone de lecture) par l'immunoglobuline antiperoxydase préparée d'une certaine façon. Par contre, si l'échantillon contient de la FSH, il y a formation de complexes IgG1 marquée à la peroxydase + FSH ; ces complexes ne sont pas reconnus par l'imunoglobuline anti-peroxydase car les diverses techniques de préparation détaillées plus loin, font que la FSH liée aux IgG1 marquées, masque les sites (de la peroxydase) choisis à l'avance comme étant les sites immunogènes reconnus par l'immunoglobuline anti-peroxydase immobilisée sur l'élément filtrant sélectif.

L'arrêt des IgG1 marquées au niveau de ce dernier élément, n'est pas spécifiquement dû à l'absence de FSH mais plutôt à la non-saturation des IgG1 reconnaissant la FSH. Les immunoglobulines immobilisées sur l'élément filtrant sélectif vont donc reconnaître et stopper toutes les IgG1 marquées à la peroxydase non saturées par leur antigène propre, les mêmes immunoglobulines immobilisées agissant ainsi quel que soit l'antigène : c'est donc une phase sélective universelle. Elle ne nécessite plus, comme avant, d'être identique au ligand recherché : Exemple : dans le dosage de la FSH, il fallait fixer de la FSH sur cette phase sélective : les anticorps marqués anti-FSH déposés sur une première couche, ne rencontrant pas de FSH dans l'échantillon, étaient stoppés par la FSH de cette phase sélective. Il fallait donc immobiliser de la FSH sur la phase sélective.

Or,le procédé objet de la présente invention, se propose de détecter plusieurs substances en même temps dans une stratégie de "pré-screening" : si au moins une des substances recherchées est présente dans l'échantillon, il convient alors de pratiquer des dosages plus ciblés.Exemple : dosage des marqueurs tumoraux de la sphère digestive (Alpha foeto protéine pour le foie,CA 19-9 pour le pancréas, Antigène carcino embryonnaire pour le colon,etc..). Si au moins un de ces marqueurs est en excès (option semi-quantitative détaillée plus loin), le procédé le révèle, orientant le médecin vers la sphère digestive pour des examens plus détaillés.

Il est donc impossible d'immobiliser tous ces marqueurs sur la même phase sélective pour stopper les anticorps marqués déposés sur la couche supérieure du dispsositif : chacun des marqueurs a son propre pK,pI,etc.. et les techniques de fixation varient d'une protéine à l'autre : certains marqueurs ne possèdent pas de résidu lysine : le même blocking ne peut pas être utilisé dans ce cas,etc..

Par contre il est possible de déposer sur la couche supérieure du dispositif une association d'anticorps marqués à la peroxydase, chacun spécifique d'un marqueur donné.Ils seront tous stoppés par la même immunoglobuline anti peroxydase, s'il n'existe aucun marqueur. S'il existe au moins un marqueur, son anticorps spécifique sera donc saturé et ne sera pas reconnu par l'immunoglobuline-anti peroxydase immobilisée sur l'élément filtrant sélectif, et migrera vers la zone de révélation.

La présente invention s'est en conséquence donné pour but de fournir un dispositif et un procédé pour la détermination qualitative et quantitative rapide de la présence d'un ligand réactif, dans un fluide, répondant mieux aux nécessités de la pratique que les dispositifs et procédés visant aux mêmes buts proposés dans l'art antérieur, notamment en ce que le moyen de sélection entre un complexe antigène + anticorps et un des ligands seul, non complexé, est plus simple et facile, en ne faisant pas intervenir directement et spécifiquement le ligand recherché, ce qui donne un caractère universel au procédé.

L'invention concerne un procédé de détermination de la présence ou de l'absence d'un ligand [X] dans un échantillon fluide à tester, ledit procédé, qui met en oeuvre un dispositif filtrant et une réaction du type antigène/anticorps :

$$X + anti(X) \Rightarrow X\text{-}anti(X) \text{ ou } E^* + anti(E^*) \Rightarrow E^*\text{-}anti(E^*)$$

où E* est une enzyme de marquage, étant caractérisé en ce que

(1°) on met en contact, dans une première zone, l'échantillon fluide à tester susceptible de contenir le ligand [X] avec un anticorps marqué par une enzyme [anti(X)-E*], pour obtenir un complexe de formule :

$$X\text{-}anti(X)\text{-}E^*$$

quand ledit ligand X est présent dans ledit échantillon,

(2°) on met en contact, dans une seconde zone, le complexe X-anti(X)-E* ainsi obtenu quand X est présent dans l'échantillon à tester, ou l'anticorps marqué anti(X)-E* quand X n'est pas présent dans ledit échantillon, avec un anticorps immobilisé spécifique de l'enzyme de marquage de structure:

$$\vdash anti(E^*)$$

où ⊢ symbolise l'immobilisation de l'anticorps anti(enzyme de marquage), ledit anticorps ⊢anti(E*) ne reconnaissant pas l'enzyme de marquage dans le complexe X-anti(X)-E* mais reconnaissant ladite enzyme de marquage dans l'anticorps marqué anti(X)-E* qui est immobilisé, selon les mécanismes :

$$\vdash anti(E^*) + X\text{-}anti(X)\text{-}E^* \Rightarrow \text{pas de réaction}$$

$$\vdash anti(E^*) + anti(X)\text{-}E^* \Rightarrow \vdash anti(E^*)\text{-}E^*\text{-}anti(X)$$

puis

(3°) on révèle, dans une troisième zone, l'enzyme de marquage E* du complexe X-anti(X)-E* au moyen d'un substrat spécifique de ladite enzyme de marquage, pour apprécier la présence dudit ligand X dans l'échantillon à tester.

D'autres caractéristiques du procédé de l'invention figurent dans les revendications secondaires 2-7 ci-après.

L'invention vise également un dispositif de détermination d'un ligand X dans un échantillon fluide à tester, caractérisé en ce qu'il comprend un système filtrant comportant

- une première zone poreuse comportant un matériau marqué non immobilisé anti(X)-E*,
- une seconde zone poreuse comportant un matériau immobilisé ⊢anti(E*), qui lors de la migration réagit avec anti (X)-E* mais ne réagit pas avec le complexe X-anti(X)-E* formé dans la première zone quand X est présent dans ledit échantillon à tester,
- une troisième zone comportant un substrat spécifique de l'enzyme de marquage pour révéler le complexe X-anti (X)-E* quand X est présent dans ledit échantillon à tester.

Selon l'invention, on fait appel à une structure comportant au moins trois zones :

- une première zone de réaction qui comprend une membrane poreuse perméable prévue pour recevoir un échantillon du fluide à tester et destinée à être associée à au moins un réactif convenablement marqué, apte à reconnaître le (ou les) ligand (s) recherché(s) éventuellement contenu(s) dans l'échantillon
- une seconde zone de réaction, dite sélective, totalement distincte et separée de la première,comprenant une phase solide poreuse perméable contenant un réactif de référence, non marqué, apte à reconnaître le (ou les) réactif(s) marqué(s) (déposé dans la première zone), mais seulement quand ce (ou ces) réactif (s) marqué(s) est saturé par son ligand, ce réactif de référence étant totalement indépendant du ligand;
- une troisième zone de réaction, dite de révélation, également poreuse et imperméable, totalement distincte et séparée de la seconde zone,contenant des moyens de révélation de la réaction qui s'est éventuellement produite dans la première ou la seconde zone de réaction.

Selon un mode de réalisation avantageux de l'invention, la première zone de réaction est propre à recevoir une phase solide avantageusement supportée par la première membrane, qui comprend au moins un réactif marqué de façon appropriée, notamment par une enzyme, un radioisotope, un produit chimique chromophore, fluorescent ou chemiluminescent,ce réactif marqué étant avantageusement un anticorps ou un antigène.

Selon un autre mode de réalisation avantageux de l'invention, la phase solide contenant un réactif non marqué qui se trouve dans la seconde zone de réaction, est constituée par un support insoluble sur lequel est fixé le dit réactif (immunoglobuline notamment) non marqué, qui correspond soit à l'enzyme de marquage des réactifs de la première zone, soit au(x) réactif(s) lui même de la dite première zone de réaction, lequel support insoluble est choisi parmi les microsphères, les microplaques, les gels (polyacrylamide, agarose, etc..) ou autres supports bien connus.

Selon un autre mode de réalisation avantageux de l'invention, la troisième zone de réaction contient un substrat chromogène quand il est prévu que la lecture de la réaction doit se faire par colorimétrie.

Selon un autre mode avantageux de réalisation il est prévu au dessus de la première zone de réaction, une zone supplémentaire destinée à neutraliser une certaine quantité seulement de ligand recherché, cette quantité correspondant à un seuil limite considéré comme normal dans l'échantillon. La quantité de ligand excédentaire, quand elle existe, traverse alors cette zone supplémentaire pour réagir avec les réactifs marqués dans la première zone décrite plus haut et plus tard avec le reste du dispositif. Ce qui permet un dosage semi-quantitatif.

Selon une autre disposition avantageuse de ce mode de réalisation, la zone supplémentaire est une membrane, avantageusement associée à un réactif (notamment un anticorps) capable de fixer (et donc de neutraliser) une certaine quantité seulement de ligand recherché, laquelle quantité correspondant à un seuil limite considéré comme normal dans l'échantillon; ce qui permet un dosage semi-quantitatif.

Selon une autre disposition avantageuse de ce mode de réalisation, le réactif non marqué destiné à neutraliser une certaine quantité seulement de ligand, peut être associé fixé, immobilisé, a la première ou à la seconde zone de réaction. Conformément à la présente invention, il est possible de réaliser des dosages quantitatifs, en associant la troisième zone de réaction à un appareillage de quantification connu en lui même, tel que RIA, spectrophotomètre, etc...

Selon un mode de réalisation avantageux du dispositif conforme à la présente invention, les zones de réaction sus dites sont superposées pour former de haut en bas : un étage supérieur contenant la première zone de réaction, un étage médian contenant la seconde zone de réaction et un étage inférieur contenant la troisième zone de réaction, et éventuellement un étage supplémentaire au dessus de l'étage supérieur, pour la zone de réaction supplémentaire dans le cas de dispositif de dosage semi-quantitatif.

Le procédé de l'invention pour la détermination qualitative et quantitative rapide de la présence d'un ligand réactif dans un fluide comprend ce qui suit. Au cours d'une première étape, un echantillon de fluide à analyser est mis en contact dans une première zone de réaction, avec un réactif marqué tel qu'un anticorps ou un antigène marqué. Au cours d'une seconde étape, le produit de la première réaction entre en contact dans une seconde zone de réaction, avec un second réactif, non marqué, possédant la particularité de reconnaître si le réactif marqué de la première zone est ou non, saturé en ligand recherché, cette reconnaissance étant totalement indépendante du ligand recherché puisque ce réactif de cette seconde zone est le même quel que soit le ligand recherché dans l'échantillon. Enfin au cours d'une troisième étape, le produit de la première ou de la deuxième réaction entre en contact dans une troisième zone de réaction, avec des moyens de révélation de la présence - ou de l'absence - du ligand que l'on cherche à détecter.

Selon un mode de mise en oeuvre du procédé conforme à la présente invention, l'échantillon de fluide est mis en contact, avant la première étape du procédé décrit ci dessus, avec un réactif non marqué destiné à neutraliser ou fixer une certaine quantité seulement de ligand recherché, correspondant à un seuil limite considéré comme normal dans l'échantillon; la quantité excédentaire de ligand, si elle existe, entre alors en contact avec les réactifs des différentes zones successives comme décrit plus haut.

Le dispositif conforme à la présente invention, representé à la Figure 1 - Planche n° 1 (Représentation schématique en coupe orthogonale d'un mode de réalisation du dispositif conforme à la présente invention) comprend de bas en haut un support A qui porte une pastille de cellulose ou autre matrice à large porosité et imprégnée d'un spot de substrat chromogène B, au dessus de laquelle est disposée une membrane C de porosité beaucoup plus serrée, imprégnée du réactif de référence non marqué. Une membrane D de porosité large, est imprégnée du réactif marqué qui reconnaitra le ligand éventuellement présent dans l'échantillon.Ce dernier sera déposé dans l'orifice E d'une plaque F identique à A, enserrant ainsi l'empilement des pastilles.

Les ligands que l'on cherche à détecter à l'aide de ce dispositif sont des antigènes ou des anticorps mais aussi toutes sortes de substances chimiques ou biologiques. Les applications sont très nombreuses ; l'on peut citer parmi elles le dépistage précoce de cancers, les dosages enzymatiques, le diagnostic d'affections virales ou microbiennes, le dépistage des maladies auto-immunes, le dosage des facteurs de croissance tels l'EGF (Epidermal Growth Factor), le FGF (Fibroblast Growth Factor),les TGF (Transforming Growth Factor $\alpha$ et $\beta$), le TNF (Tumor Necrosis Factor), les IGF (Insuline-like Growth Factor), le PDGF (Platelet Derivated GF), les CSF (Colony Stimulating Factor), etc... tous plus ou moins impliqués dans des processus néoplasiques, le dosage des cytokines (Interleukines 1 à 7,interféron $\alpha$,

β et gamma,érythropoïétine,etc), le dosage des récepteurs membranaires de ces facteurs de croissance et de ces cytokines : le taux de ces récepteurs membranaires est multiplié par 100 ou 1000 dans de nombreux cancers, le dosage d'hormones mais surtout le dosage des récepteurs de ces hormones car il est très récemment apparu qu'il existait des relations entre le taux de récepteurs de ces hormones et certains cancers (prostate pour la testostérone, sein pour les oestrogènes,etc) et qu'une association excès de récepteurs aux facteurs de croissance + effondrement des récepteurs de l'oestrogène était de mauvais pronostic dans le cancer du sein par exemple et impliquait un traitement hormonal en plus des traitements chirurgicaux et ionisants, le dosage des leucotriènes et des prostaglandines dans les processus inflammatoires graves, le dosage de leurs récepteurs, le dépistage d'allergies (IgE), le dosage de neuromédiateurs, des récepteurs de ces neuromédiateurs (effondrés dans de nombreuses pathologies de sénilité cérébrale) surtout depuis la découverte du troisème récepteur de la dopamine (D3) qui se révèle être la véritable cible à traiter dans les psychopathies graves alors que les traitements antérieurs visaient à bloquer les récepteurs D1 et D2 avec le cortège bien connu d'effets secondaires graves (contractures musculaires, ébriété, etc), inexistants quand on bloque le récepteur D3,le dosage de médicaments,etc.. Cette énumération n'est pas exhaustive ni limitative;elle veut simplement mettre en évidence le grand nombre d'examens,autrefois compliqués et coûteux, rendus faciles et abordables par le procédé objet de la présente invention, pour le bénéfice d'un dépistage systématique et précoce d'affections graves.

La Figure 2 - Planche n° 1/6 montre les différents réactifs du procédé :

- les réactifs marqués (1) qui reconnaissent le ligand à détecter sont essentiellement des anticorps ou des antigènes, déposés à l'étage supérieur sur la membrane D, en solution dans un mélange complexe décrit plus loin et visant à la conservation et à la stabilité du matériel de marquage surtout (enzymes sensibles à la lumière et à l'oxygène de l'air par exemple, soit une substance fluorescente, un radio-isotope, tout produit chimique susceptible de donner une réaction colorée avec un substrat approprié),
- les réactifs (2) de l'étage médian C sont de préférence mais pas exclusivement des immunoglobulines qui piègent, mais uniquement en absence de ligand dans l'échantillon, les réactifs marqués de l'étage supérieur, de plusieurs façons différentes ou combinées :
- soit en piégeant l'enzyme de marquage (dans les procédés colorimètriques) toujours en absence de ligand,par l'intermédiaire d'une imunoglobuline anti-enzyme spécifique de cette enzyme,
- soit en piégeant le réactif marque quand c'est un anticorps, par l'intermédiaire d'une immunoglobuline anti-Fab spécifique des sites NH2 des anticorps marqués de l'étage supérieur, sites libres puisqu'il n'y a pas de ligand dans l'échantillon. Si par contre il existe un ligand dans l'échantillon, les sites Fab de l'anticorps marqué de l'étage supérieur, sont saturés en ligand et ne seront pas reconnus par les immunoglobulines anti-Fab,
- soit en piégeant les deux : l'enzyme de marquage et l'anticorps marqué,toujours en l'absence de ligand dans l'échantillon, par l'intermédiaire d'une association d'immunoglobulines : une anti-enzyme et une anti-Fab. L'absence de ligand rend ces deux derniers sites accesssibles aux immunoglobulines dirigées contre eux. Tandis que la présence de ligand, vient masquer l'enzyme (sans l'inhiber pour autant) et occupe les sites Fab des anticorps marqués de l'étage supérieur.

Ces immunoglobulines de l'étage médian jouent donc un rôle capital et ceci quel que soit le ligand, ce qui fait leur caractère universel. Il faut néanmoins préciser que les anticorps marqués de l'étage supérieur (dans le cas de dosages d'antigènes), doivent :

- être de la même origine (ex : IgG1 de souris) pour que les immunoglobulines (ex : anti Fab d'IgG1 de souris) de l'étage médian les reconnaissent sans différence, quel que soit le ligand à doser ;
- porter le même marquage ( ex: peroxydase) pour que les immunoglobulines (ex : anti-peroxydase) de l'étage médian les reconnaissent tous et les piègent, sauf si l'antigène recherché vient masquer les molécules de peroxydase et empêche ainsi les immunoglobulines anti-peroxydase de reconnaître la peroxydase portée par les anticorps marqués. Ces derniers traversent alors cette phase sélective pour aller colorer le substrat en B;
- être marqués d'une certaine manière : il faut que l'enzyme soit fixée le plus près possible des sites Fab des anticorps de l'étage supérieur : de telle sorte que la présence d'un ligand volumineux comme par exemple un récepteur membranaire (400 kD), masque complètement l'enzyme (sans pour autant l'inhiber) : l'utilisation d'anticorps marqués F(ab)'2 est donc préférable aux anticorps entiers.

Ces immunoglobulines de l'étage médian peuvent être avantageusement supportées par des microsphères (latex, agarose, polystyrène, polyarylamide, gels divers, etc...) portant un réactif, destiné à retenir les réactifs marqués restés libre à l'étage supérieur.

Dans ces conditions, si l'on recherche un antigène dans une goutte de fluide biologique, en cas de réaction positive, l'antigène présent dans le fluide se lie à l'anticorps marqué contenu dans la pastille D ; le fluide contenant le complexe antigène-anticorps marqué précité traverse la couche de microsphères déposées en C, sans être retenu par ces dernières puis parvient sur la membrane B de révélation contenant un substrat qui est ainsi coloré par contact avec le fluide examiné.

En cas de réaction négative, c'est à dire si l'échantillon examiné ne contient pas d'antigène, l'anticorps marqué de l'étage supérieur est retenu par le réactif non marqué fixé sur les microsphères en C, et ne vient pas colorer le substrat en B.

Les modalités sont les mêmes, mutatis mutandis, si c'est un anticorps que l'on recherche dans le fluide.

Si on veut obtenir un dosage semi-quantitatif (Figure 3 - Planche n° 1), une pastille supplémentaire appelée C0, placée au dessus de la pastille D, immobilise et neutralise par l'intermédiaire de réactifs appropriés (3), une certaine quantité seulement de ligand recherché, correspondant à une quantité considérée comme normale dans le fluide. La quantité excédentaire de ligand, si elle existe, traverse cette membrane C0 pour réagir normalement avec les réactifs des zones décrites plus haut.

La Figure 4 - Planche n° 2/6 illustre le dosage qualitatif d'antigènes avec obtention d'un résultat positif : on y voit le complexe (5) antigène-anticorps marqué formé traverser l'étage médian sans être retenu, pour aller vers la zone de révélation B tandis que les anticorps restant (6) non saturés en antigènes sont piégés en C.

La Figure 5 - Planche n° 2/6 illustre le dosage qualitatif d'anticorps avec obtention d'un résultat positif : les antigènes marqués (7) saturent les anticorps sériques recherchés et traversent l'étage médian tandis que les antigènes marqués restés libres (8) sont piégés en C.

EXEMPLE 1 : DOSAGE DU EGF-R (RECEPTEUR DE L' EPIDERMAL GROWTH FACTOR )

1 - Les supports

En B : Papier Durieux No 122
En C : Nylon Pall® (porosité 0.45 µm épaisseur 100 µm)
En D : Papier Whatman® traité au préalable par du lait écrémé 5%

2 - Les réactifs

En B : un spot de 2 µl de la solution suivante :

* 3-3'-5-5' Tétra Méthyl Benzidine à 1 mg/ml
* Tampon Citrate 0,01 M

En C : Anticorps Goat Anti-Peroxydase (Sigma, Ref P 5774)
En D : Monoclonal Anti EGF - Receptor : Clone No 29.1.1. Mouse IgG1

Marqué Peroxydase. (Yarden et al. J.BIOL.CHEM..,260,315 (1985))
Déposé en solution de D-Glucose 0,1 M in PBS

3 - Mode opératoire proprement dit

3 a - Confection des pastilles et technique de marquage des anticorps :

- 1 : Dissoudre 30 mg de PBQ (p - benzoquinone) dans 1 ml d'ethanol.
- 2 : Diluer 1 mg d'anticorps Monoclonal Goat Anti-Receptor dans 0,15 ml de NaCl 0,15 M contenant 0,05 ml de tampon phosphate 1 M à pH 6.
- 3 : Ajouter 0,05 ml de la solution de PBQ de 1) à la dilution d'anticorps de 2).Incuber 1 heure au noir et à température ambiante.
- 4 : Eliminer l'excès de PBQ par dialyse exhaustive (une nuit).
- 5 : Ajouter 4 mg de Peroxydase. Agiter au vortex quelques minutes.
- 6 : Ajouter 0,1 ml de tampon Carbonate $NaHCO_3$ 1 M pH 9 et incuber 48 heures à 4°C.
- 7 : Dialyse extensive (une nuit) contre PBS à travers filtre Millipore 0,22 µm. Conserver au noir et à 4°C. Les pastilles Whatman sont baignées dans un bain de lait écrémé à 5 % puis rincées et séchées.

Sur chaque pastille on dépose 5 µl de la solution d'anticorps marqués obtenus, dilués à 1/1000 dans du PBS. Séchage sous vide poussé puis stocker sous vide,au noir et à 4°C.

3 b : La phase sélective :

La pastille de nylon Immunodyne Pall (0,45 µm de porosité), est plongée pendant une heure dans un bain de la solution suivante :

- Anticorps Goat Anti-Peroxydase 1 microgramme / ml
- PBS

Après une heure, rincer avec PBS glacé puis pratiquer un blocking par bain dans la solution suivante pendant une heure :

- Bovine Serum Albumine 2%
- PBS glacé.

Puis rincer plusieurs fois avec la solution suivante :

- Thimerosal 0,01 %
- Tris Buffer Saline (TBS) pH 7,4

Sécher et garder sous vide sauf deux pastilles pour le contrôle de qualité suivant :
Contrôles de qualité : Figure 6 - Planche n° 3/6

- On prend une pastille de phase sélective et on la baigne dans une solution d'anticorps anti - EGF-R, marqués à la peroxydase : Pastille T-
- On prend une seconde pastille de phase sélective et on la baigne dans une solution en excès de EGF-R (Récepteurs EGF) et d'anticorps anti-EGF-R : Pastille T+

Pendant ces bains de 15 minutes on prépare les pastilles temoins :

- Deux pastilles de Nylon sont préparées exactement de la même façon que la phase sélective sauf qu'il n'y a pas d'anticorps anti-peroxydase.Elles ne doivent rien retenir normalement : Pastilles TT.

L'une des pastilles TT est baignée en compagnie de la pastille T- dans une solution d'anticorps marqués anti EGF-R. L'autre est baignée dans la solution d'anticorps marqués anti EGF-R saturés en EGF-R.

Rinçage et séchage puis on dépose sur chaque pastille 5 µl d'une solution de chromogène suivante :
( TMB 10 mg/ml,H2O2 0,01 volume,Tampon citrate 0,1M)

La Figure 6 - Planche n° 3/6 illustre les résultats obtenus :

- Dans le bain de la solution d'anticorps marqués anti EGF-R, la pastille T- a fixé l'anticorps anti EGF-R marqué à la peroxydase qui colore la solution chromogène déposée, en bleu ; la pastille TT reste incolore : elle n'a rien fixé du tout, il n'y a pas d'anticorps anti EGF-R marqués à la peroxydase qui s'y fixent, il n'y a donc pas de rétention parasite.
- Dans la solution d'anticorps marqués anti EGF-R saturés en EGF-R, la pastille T+ n'a pas pu fixer ces anticorps : à cause de l'antigène lié à eux, leur peroxydase est devenue inaccessible aux anticorps anti peroxydase préalablement fixés sur la pastille phase sélective, ils ont été éliminés au rinçage.La dépose de la solution chromogène n'a rien coloré. La pastille temoin TT n'a rien fixé du tout également.

4 - Mode d'exécution

1 ml de PBS est versé dans 2 tubes en polypropylène numérotés 0 et 1.

Dans le tube 1 on ajoute 2 µl d'une solution de récepteurs membranaires de l'EGF obtenus par traitement de membranes de cellules de carcinome mammaire (détergent, centrifugations successives pour éliminer les noyaux, les membranes, etc... puis dialyse extensive de 24 heures et passage sur colonne Sephadex).

50 µl du tube 0 sont déposés dans l'orifice E du dispositif (D0).

50 µl du tube 1 sont déposés dans l'orifice du dispositif (D1).

RESULTATS :

Après 30 secondes on retourne les dispositifs. Le spot de TMB de D1 est bleu tandis que celui de DO reste incolore. : (Figure 7 - Planche n° 3/6)

EXEMPLE 2 : DOSAGE QUALITATIF DE L'HCG (Human chorionic gonadotropin)

Les suports,les réactifs de B (spot TMB) et C (phase sélective d'anticorps anti-peroxydase) sont identiques à l'exemple 1.

Seule la pastille D est différente : elle contient des anticorps IgG1 de souris marqués à la peroxydase, spécifiques de la sous unité β-HCG, à raison d'un spot de 5 µl contenant 5 nanogrammes de ces anticorps.

Mode d'exécution

Cinq tubes numérotés 0 à 4 contiennent une solution d'HCG in PBS à concentration croissante exprimée en Unités Internationales:
0 - 12,5 - 25 - 50 - 100 U.I./litre de PBS

Cinq dispositifs de dosage qualitatif d'HCG sont numérotés :
D0 - D12,5 - D25 - D50 - D100

On dépose 50 µl du tube 0 sur le dispositif D0, 50 µl du tube 1 sur le dispositif D12,5, 50 µl du tube 2 sur le dispositif D25, 50 µl du tube 3 sur le dispositif D50, 50 µl du tube 4 sur le dispositif D100.

On attend 30 secondes l'écoulement total des fluides et on retourne le dispositif pour la lecture du signal de coloration du TMB,signal évalué 0 à +++ :

| Résultats : | | | | | |
|---|---|---|---|---|---|
| No du Tube | 0 | 1 | 2 | 3 | 4 |
| Concentration en UI/l | 0 | 12,5 | 25 | 50 | 100 |
| Signal | 0 | + | ++ | +++ | +++ |

EXEMPLE 3 : DOSAGE SEMI-QUANTITATIF DE L'INTERLEUKINE-1

Les supports sont identiques à l'exemple 1.

Les réactifs en B (spot TMB) et en C (anticorps anti peroxydase) sont identiques aussi.

En D : Anticorps " Rabbit Anti Human Interleukine 1β "obtenus de Genzyme Corp. (Koch Light Ltd., England) marqués à la peroxydase. En C0 : Membrane de Nylon Pall® de porosité 0,45 microns, de 100 microns d'épaisseur traitée par un bain d'une solution très diluée (0,1 µg/ml) d'anticorps anti IL-1β cité ci-dessus, puis rincée et saturée par BSA 5 % et PBS glacé et encore rincée pour éliminer la BSA en excès.Sécher et conserver à 4°C. Cette membrane C0 va piéger l'IL-1β contenue dans l'échantillon à analyser à hauteur de 505 picogrammes par ml, c'est-à-dire 5 UI d'IL-1β/ml.La quantité d'IL-1β excédentaire, si elle existe,va traverser C0 et se lier aux anticorps marqués en D puis traverser C sans se lier aux anticorps anti-peroxydase, et arriver enfin en B pour la révélation : coloration du TMB. Tandis que si le taux d'IL-1β est inférieur ou égal à 5 UI/ml dans l'échantillon, les anticorps non marqués immobilisés en C0 piègent l'IL-1β tandis que les anticorps marqués anti IL-1β présents en D, ne reçoivent pas d'IL-1β et migrent avec le fluide de l'échantillon vers le bas, vers C où ils sont piégés par les anticorps anti peroxydase. Il n'y aura pas d'anticorps marqués peroxydase en B et donc pas de coloration.

La Figure 8 - Planche n° 4/6 : illustre un dosage semi-quantitatif d'IL-1β négatif à droite (IL-1β $\leq$ 5 UI/ml) et positif à gauche (IL-1β $\geq$ 5 UI/ml)

EXEMPLE 4 : DOSAGE SEMI-QUANTITATIF DE L'ANTIGENE CARCINOEMBRYONNAIRE

Mêmes supports. Mêmes réactifs en B (Substrat chromogène)

Reactifs :

- En C : Goat Anti Mouse IgG Fab specific (Ref M 6898, Sigma)
- En D : Anticorps monoclonaux Mouse anti-ACE marqués à la peroxydase : Ref 69.186.1 de Miles Lab.
- En C0 : Anticorps anti ACE non marqués, immobilisés sur Nylon

MODE OPERATOIRE :

Préparation de la membrane C0

Une solution très diluée d'anticorps anti-ACE (0,1 µg/ml) sature,selon le protocole Pall, une membrane en nylon de type Immunodyne 0,45 µm de porosité et 100 µm d'épaisseur. Après une agitation douce d'une heure, rinçage et bain de blocking (BSA 5 %,PBS) puis trois rinçages au PBS glacé et séchage. Conserver à sec et au noir,sous vide.

Cette pastille C0 ainsi préparée piège l'ACE (2,5 nanogrammes/ml) considéré comme seuil limite dans le sang d'un adulte normal (non fumeur et femme non enceinte).

Préparation de la membrane D (Anticorps marqués)

La solution d'anticorps Mouse anti-ACE, marqués à la peroxydase est diluée à 10 µg/ml dans une solution de PBS et de D-Glucose à 5 %.On dépose 5 µl de cette solution sur les pastilles de papier Whatman préalablement saturé par du lait écrémé à 5 %.

Préparation de la membrane C (Phase sélective)

Les pastilles de nylon Pall® Immunodyne sont baignées pendant une heure à température ambiante dans la solution d'anticorps de Goat anti Mouse IgG Fab diluée à 1 µg/ml de PBS. Puis rinçages et blocking comme décrit plus haut, puis rinçage final et conserver à 4°C au noir et sous vide. Cette membrane piège les anticorps marqués, Mouse anti-ACE, quand ils ne sont pas saturés en ACE car les anticorps anti-ACE sont issus de souris et les anticorps de cette phase sélective ne reconnaissent que la partie Fab des anticorps de souris : Mouse anti-ACE, libres de tout ACE ; l'ACE excédentaire (au dessus de 2,5 ng/ml de sérum) dans l'échantillon va se lier aux sites Fab des anticorps marqués Mouse anti-ACE, et ces sites Fab vont se trouver masqués par l'ACE et ne seront donc pas reconnus par les anticorps anti Mouse Fab fixés dans la membrane C. Les complexes Anticorps marqués anti-ACE + l'ACE excédentaire qui a débordé la membrane C0, vont traverser cette phase sélective C pour migrer avec le fluide de l'échantillon vers la zone de révélation B.

Tandis que les anticorps marqués Mouse anti-ACE restés libres de tout ACE vont être piégés en C par les anticorps anti Mouse Fab.

MODE D'EXECUTION :

Six tubes de polypropylène sont numérotés 0 - 1 - 2 - 3 - 5 - 10.

Ils contiennent de l'ACE dans PBS aux concentrations suivantes : Tube 0 : Témoin sans ACE.

Tube 1 : 1 ng d'ACE/ml -- Tube 2 : 2 ng/ml -- Tube 3 : 3 ng/ml Tube 5 : 5 ng/ml -- Tube 10 : 10 ng/ml

Six dispositifs semi-quantitatifs sont numérotés D0 - D1- D2 - D3 - D5 - D10.

50 microlitres du tube 0 sont déposés dans le dispositif D0 50 µl de 1 dans D1, 50 µl de 2 dans D2, 50 µl de 3 dans D3, 50 µl de 5 dans D5, 50 µl de 10 dans D10.

On attend 30 secondes l'écoulement complet des fluides des échantillons puis on retourne les dispositifs pour la lecture de la coloration du spot de TMB :

| Résultats : | | | | | |
|---|---|---|---|---|---|
| No du tube | | 0 | 1 | 2 | 3 |
| 5 | 10 | | | | |
| (Taux ACE en ng/ml) | | | | | |
| - SIGNAL | | 0 | 0 | 0 | + |
| ++ | +++ | Noté 0 à +++ | | | |
| - | | | | | |

EXEMPLE 5 : DOSAGE QUANTITATIF RADIO-ACTIF DU CA 125 : Figure 9 - Planche n° 5/6

Le CA 125 est un marqueur tumoral unique pour le diagnostic et le suivi de cancers gynécologiques et en particulier de carcinome de l'ovaire. Il est associé à une glycoprotéine de haut poids moléculaire qui n'existe pas dans les ovaires normaux mais qui est retrouvée en abondance dans les adénocarcinomes ovariens.

Le taux de concentration sérique ou plasmatique du CA 125 des sujets normaux est de $8{,}7 \pm 8{,}9$ Unités/ml. 99 % de la population normale a des concentrations de CA 125 $\leq$ 35 U/ml. L'Unité correspond à un certain poids défini de glycoprotéine portant le déterminant antigènique CA 125 (environ 1 nanogramme) Le dispositif utilisé va comprendre de haut en bas : Figure 9 - Planche n° 5/6

- la plaque F avec son orifice E pour déposer l'échantillon

- la membrane D portant des anticorps Mouse IgGI (fragment Fab) anti CA 125 marqués radioactivement
- la membrane C portant des anticorps anti Mouse IgG1 fragment Fab
- une épaisse membrane perméable M, poreuse, de cellulose saturée par de la caséine à 5 % pour ne pas retenir de substances et destinée à établir une séparation entre la phase sélective C et la membrane de lecture B : ce qui permet au compteur placé sous B de ne pas entrer en contact avec les anticorps radioactifs piégés en C, et donc d'éviter des faux positifs
- la phase de lecture B : une membrane en nylon qui absorbe le produit des réactions en C et D : c'est à dire qui absorbe les éventuels complexes anticorps marqués radioactivement + l'antigène recherché. Cette membrane B est apte à entrer en contact intime avec le capteur d'un lecteur ou d'un compteur de radioactivité, pour évaluer l'intensité du signal en fonction du nombre des anticorps marqués radioactifs, complexés à leur antigène qui parviennent en B.
- La plaque transparente A est à son tour perforée d'un orifice dans lequel viendra s'enfoncer le capteur du compteur de radioactivité.

MODE OPERATOIRE

Membrane D : 10 mm de diamètre,en papier Durieux N°122 baigné quelques heures dans une solution de lait écrémé à 5 % dans de l'eau déionisée puis rincé au PBS et séché.

Le lendemain, on note la face de dépose de ces membranes par un petit trait au crayon noir et on dépose 5 µl de la solution suivante :

- Anticorps Mouse IgG1 F(ab')2 Anti-CA 125 marqués à l'Indium 111 : 1 microgramme par ml
  (Ref OC 125, CI Saclay)
- Sucrose 5 %
- Thimerosal 0,01 %
- NaCl 0,9 %
- Tampon acétate 0,1 M pH 5

(Les 50 µl de l'échantillon vont diluer ces 5 µl de tampon jusqu'à → 0,01M)

Séchage sous vide poussé à froid et stockage au froid en attendant l'assemblage avec les autres membranes

Membrane C . en nylon Pall 0,45 µm de porosité et 100µm d'épaisseur, 10 mm de diamètre, baignée quelques heures dans de la solution suivante :

- Anticorps Goat Anti Mouse IgG1 F(ab')2 spécifique (Ref Signa 6898) dilués à 1 microgramme par ml de PBS puis rinçage et blocking avec bain de BSA 5 % dans PBS 1 heure à 37°C puis rinçage final au PBS glacé,séchage et stockage au froid en attendant l'assemblage des diverses membranes.

Membrane M : la préférence est allée à la fibre de verre Whatman® d'épaisseur 0,25 mm baignée pendant 3 heures dans de la caséine à 5 % dans TRIS pH 6 puis rincée avec PBS plusieurs fois. Ainsi préparée, elle constitue une excellente séparation entre C et B, sans pour autant retenir anormalement les éventuels complexes qui la traversent.

Membrane B : Nylon NYTRAN® de Schleicher et Schull sans la moindre préparation : elle absorbe les éventuels anticorps radioactifs qui ont échappé à C et traversé M. Elle est apte à venir en contact avec un compteur radioactif de coups par minute (cpm sur la Figure 9 - Planche n° 5/6)

Mode d'exécution : En regard de l'orifice de la plaque transparente A on dépose dans l'ordre :

- la membrane B
- la pastille de fibre de verre M
- la membrane C
- la membrane D
- en regard de D,la plaque F est soudée à l'ensemble et perforée de l'orifice E dans lequel on déposera l'échantillon.

On prend 8 tubes numérotés de 1 à 8 . Ils contiennent le sérum provenant de la même patiente chez laquelle a été découvert un cystadénocarcinome ovarien.Ils montrent l'évolution du CA-125

- Tube 1 : jour du diagnostic
- Tubes 2,3,4 : au cours des 5 mois de la chimiothérapie
- tube 5 : à la fin de la radiothérapie

- tube 6 : 2 mois après la radiothérapie
- tube 7 : 3 mois après la radiothérapie
- tube 8 : 6 mois après la radiothérapie

On prend 8 dispositifs de dosage radioactifs de CA 125 et on les numérote D1 à D8.

50 µl du tube 1 sont ainsi déposés sur le dispositif D1, 50 µl de 2 sur D2, 50 µl de 3 sur D3,etc..

On attend chaque fois 30 secondes et on insère le dispositif dans la fente d'un appareillage de lecture de radioactivité étalonné à cet effet (1 U de CA 125 correspondant à un certain nombre de coups par minutes)

L'insertion du dispositif doit se faire de telle sorte que la membrane B soit au contact étroit avec le capteur du compteur, de la même façon qu'une carte magnétique de téléphone ou une carte bancaire.

Les résultats sont notés et transposés sur une courbe : Figure 10 - Planche n° 6/6

| N° du tube | Correspond à : | Signal en U/ml | |
|---|---|---|---|
| 1 | Découverte tumeur | | 390 |
| 2 | 3 mois de chimiothérapie | 80 | |
| 3 | 4 mois de chimiothérapie | 31 | |
| 4 | 5 mois de chimiothérapie | 7 | |
| 5 | 4 mois de radiothérapie | | 2 |
| 6 | 2 mois sans soins | | 37 |
| 7 | 3 mois sans soins | | 59 |
| 8 | 6 mois sans soins:rechute | | 495 |

<u>EXEMPLE 6 : DOSAGE SEMI-QUANTITATIF DE PLUSIEURS MARQUEURS TUMORAUX AVEC LA MEME GOUTTE D'ECHANTILLON</u> : CA 19-9 + CA 15-3 + ACE

Le principe : Pré-screening : Si au moins un des marqueurs est très augmenté,le test se colorera en bleu. Il conviendra alors d'orienter les recherches vers l'un des marqueurs pris isolément comme dans l'exemple 4.

Ce dosage est très important pour le dépistage et le suivi de cancéreux opérés pour prévenir rapidement les métastases : les taux d'ACE ou de CA 19-9 reviennent en quelques mois à la normale après la chirurgie et l'irradiation des tumeurs. Par contre les récidives peuvent être silencieuses et invisibles même avec des examens techniques sophistiqués (la fibroscopie digestive ne peut déceler des microtumeurs récidivantes,et quand ces dernières sont importantes, il est souvent trop tard ; alors que le dosage des marqueurs en association est capable de dépister de telles récidives plus tôt).

Le CA 15-3 est décrit dans la littérature comme étant un antigène associé au cancer du sein et défini par deux anticorps monoclonaux différents : 115 D8 (Hilkens et al. Int. J. Cancer (1984) 34 : 197-206) et DF3 (Kufe et al. Hybridoma (1984) 3 : 223-232). Le taux de concentration en CA 15-3 dans le sérum ou le plasma de sujets normaux est de 13,7 ± 5,2 Unités / ml. 99,5 % de la population normale a une concentration de CA 15-3 ≤ 30 U/ml.

Le CA 19-9 est le déterminant antigènique associé aux tumeurs du pancréas : c'est un dérivé sialylé du pentasaccharide du groupe sanguin LEWIS A (Lacto-N-fucopentaose-II). Ce déterminant antigènique qui se répète est porté soit par un ganglioside à la surface des membranes cellulaires soit par une protéine de type mucine dans le sérum. 1 Unité est = à 0,8 nanogramme de glycoprotéine de type mucine portant le déterminant antigènique CA 19-9.
Le taux de concentration du CA 19-9 dans le sérum ou le plasma de sujets normaux est de 8,4 Unités/ml ± 7,4 Unités/ml. 99,5 % de la population normale a des concentrations de CA 19-9 ≤ 37 Unités/ml.

Les marqueurs tumoraux présentent un inconvénient important : ils peuvent être augmentés chez certaines personnes non cancéreuses, et peuvent être à des taux normaux chez des cancéreux. Par contre, pris en association, leur spécificité est plus nette.

Exemple : cancers gastriques : (H.J.Staab - Tübingen - Germany)

- Augmentation du CA 19-9 dans : 76 % des cas
- Augmentation de l'ACE dans : 59 % des cas
- Augmentation ACE+CA 19-9 : 94 % des cas

Autre exemple : Adénocarcinome du sein :
(May-Levin et ai. 3e Congrès International Hormones et Cancer. Hambourg. RFA, 6-11 Septembre 1987 : "Etude comparative à différents stades du cancer du sein")

- sans métastases : Augmentation du CA 15-3 dans 30 à 50 % des cas du CA 15-3 et ACE dans 90 % des cas
- avec métastases : Augmentation du CA 15-3 dans 75 % des cas du CA 15-3 et ACE dans 95 % des cas Il est impossible de fixer en C (la phase sélective) tous les antigènes identiques à ceux que l'on cherche à détecter (Brevets antérieurs), pour piéger les anticorps marqués provenant de D non saturés en antigènes : pour plusieurs raisons :
- Une association de CA 15-3,CA 19-9 et ACE fixés en C, entraîne un encombrement stérique préjudiciable à la reconnaissance de leurs sites antigèniques par les anticorps marqués provenant de B; ces derniers risquent donc de ne pas être piégés et d'aller colorer B, en faux positif.
- Cette gêne n'existe pas avec des immunoglobulines différentes,car fixées par leur extrêmité Fc et convenablement orientées vers l'antigène recherché (ce qui permet leur utilisation en C0 pour neutraliser la quantité d'antigène établie à l'avance).
- Les antigènes sont rares et coûteux alors que leurs anticorps ne le sont pas.
- Leur conservation sur la phase sélective est aléatoire car leur structure tertiaire se modifie alors que les immunglobulines en général ont une aptitude extraordinaire de stabilité même à sec.

<u>MODE OPERATOIRE</u> :

Les supports, la membrane B (TMB) sont les mêmes qu'à l'exemple 1.

La membrane D va supporter les anticorps monoclonaux marqués à la peroxydase différents spécifiques de chaque antigène recherché :

- Anticorps DF3 (Cie Oris Industrie) Anti CA 15-3
- Anticorps Anti ACE (Abbott CEA EIA Monoclonal)
- Anticorps NS 116 Anti CA 19-9 (International CIS)

  - tous provenant de souris et appartenant à la catégorie d'immunoglulines IgG1
  - tous marqués à la peroxydase sur leur segment Fab
  - tous dilués à 1 microgramme par ml dans du PBS, thimerosal 0,01 % et D-Glucose 5 %

La membrane C (phase sélective) va suporter les deux types d'immunoglobulines citées plus haut dans l'exemple 4 (dosage ACE)

- Goat Anti Fab de Mouse IgG1
- Goat Anti-Peroxydase

Cette association s'est révélée très intéressante car beaucoup plus efficace : les anticorps marqués provenant de la membrane D,non saturés en antigène (marqueur par exemple), sont fixés beaucoup plus efficacement par leur fragment Fab resté libre et par l'enzyme qu'ils supportent, non masqué par l'antigène.

Alors que ce dernier, quand il se lie aux anticorps marqués, masque l'enzyme et le site Fab.

Les membranes C sont baignées pendant deux heures dans une solution des deux anticorps cités dans le PBS puis rincées dans du PBS glacé et saturées en blocking comme décrit plus haut (Bain de BSA 5% puis rinçages et séchage) ; stockage à l'abri de la lumière et au froid.

- Les membranes C0, sont baignées dans une solution contenant les anticorps non marqués dirigés contre les trois marqueurs, et diluée à 0,1 microgramme par ml pour chaque anticorps. Cette membrane va piéger jusqu'à :

  - 30 U/ml de CA 15-3
  - 37 U/ml de CA 19-9
  - 7 ng/ml d'ACE

Si le taux de l'un de ces marqueurs excède ces- limites,la quantité excédentaire de marqueur va traverser la membrane C0 et se conjuguer aux anticorps marqués de D, traverser C avant d'aller colorer B.

<u>Confection du dispositif</u>

Sur une plaque transparente A on dépose :

- une pastille de substrat : B

- une pastille de phase sélective : C
- une pastille d'anticorps marqués mélangés : D
- une pastille de pré-phase sélective C0 d'anticorps dirigés contre une certaine quantité seulement des trois marqueurs. Sur cet ensemble, on dépose et soude une plaque F perforée d'un orifice E en regard des pastilles, et dans lequel seront déposés les échantillons.

Mode d'exécution

On prend quatre tubes numérotés 0 à 3. Ils contiennent :

- Tube 0 : les trois marqueurs en quantité normale :
  - CA 15-3 : ≤ 30 Unités/ml (Oris)
  - CA 19-9 : ≤ 37 U/ml (Int Cis)
  - ACE (Ref C 7292 Sigma) : ≤ 7 ng/ml
- Tube 1 : seul l'ACE est > à 7 ng/ml
- Tube 2 : seul le CA 19-9 est > à 37 U/ml
- Tube 3 : seul le CA 15-3 est > à 30 U/ml

On prend quatre dispositifs de dosage semiquantitatif des trois marqueurs tumoraux avec la même goutte et on les numérote D0 à D3 :

On dépose 50 µl du tube 0 sur le dispositif DO, 50 µl du tube 1 sur le dispositif D1, 50 µl de 2 sur D2, 50 µl de 3 sur D3. On attend 45 secondes (au lieu de 30 secondes pour les dispositifs précédents en raison de la membrane C0) et on retourne les dispositifs:

| RESULTATS | | |
|---|---|---|
| N° du tube | Marqueur en excès | SIGNAL |
| 0 | aucun | 0 |
| 1 | ACE | ++ |
| 2 | CA 19-9 | ++ |
| 3 | CA 15-3 | ++ |

Cet exemple n'est pas limitatif : étant donnée la faible quantité d'anticorps non marqués en C0 ou ceux marqués utilisés en D ( 5 nanogrammes de chaque), il s'est avéré possible d'associer plusieurs types d'anticorps spécifiques de plusieurs marqueurs, ce qui permet d'aboutir à un test universel si on utilise les marqueurs des cancers les plus répandus, et donc à un dépistage de masse.

Il s'est également révélé possible d'associer non seulement des anticorps de marqueurs entre eux, mais également des anticorps de plusieurs facteurs de croissance,mais également aussi des anticorps de marqueurs avec des anticorps de facteurs de croissance,ou des anticorps de récepteurs ou enfin une association de ces anticorps.

Exemples de dispositifs :

1 - Cancers digestifs :

EGF (Epidermal Growth Factor)+ TGF (Transforming Growth Factor) + Marqueurs ACE + AFP + CA 19-9

L'excès de l'un de ces marqueurs dans la goutte d'échantillon,provoque la coloration en B et doit faire orienter vers des examens plus ciblés sur l'appareil digestif.

2 - Cancers du sein :

EGF-R (Récepteurs de l'EGF) + PR (Récepteurs de la Progestérone) + ER ( Récepteurs de l'Oestrogène) + CA 15-3 + ACE

Une augmentation du taux des récepteurs EGF-R associée à une augmentation de marqueurs,est de mauvais pronostic.

3 - Suivi des nouveaux protocoles de traitements par TIL (Tumor Infiltrating Lymphocytes) dopés à l'interleukine-2 : la réponse optimale est une élévation de :

IL-1 (Interleukine 1) + TNF (Tumor Necrosis Factor) Dans cet exemple,c'est le signal positif semi-quantitatif (IL-1 et TNF au dessus d'un certain seuil) qui est favorable.

De même dans tous les dosages semi-quantitatifs où c'est le déficit en certains ligands, qui est pathologique :

- l'insuline
- le glucagon
- les complexes vitaminiques
- etc...

Le dispositif utilisé pour tous ces exemples se présente sous l'aspect d'une carte enserrant les diverses membranes du procédé mais la succession de ces membranes peut très bien s'insérer dans d'autres structures comme par exemple au fond des puits des plaques ELISA, ou bien cette succession de membranes peut se faire de manière horizontale : la migration des fluides de l'échantillon ne se faisant plus de haut en bas mais d'une extrémité à l'autre d'une bandelette par exemple. Tous ces exemples de dosage et d'une manière générale toutes les associations de dosage dans le même test, ne sont que des exemples illustrant les facilités apportées par le procédé faisant l'objet de la présente invention. Les combinaisons peuvent être illimitées et en fait, les divers dispositifs à venir ne seront que le reflet de la nécessite d'un préscreening pour un dépistage massif et régulier de maladies malignes débutantes.

Il faut enfin signaler "la" protéine du cancer appelée CP ("Cancer Procoagulant". Stuart G.Gordon, Barbara A. Cross. Cancer Research 550, 6229-6234, October 1st, 1990) : c'est une cystéine protéinase exprimée par les cellules cancéreuses (impliquée dans les coagulations anormales observées au début de nombreux cancers) mais jamais par les cellules normales, ce qui lui donne une très haute spécicifité (90 %) alors que la spécificité de l'ACE est basse (50 %) du fait qu'il est sécrété par bon nombre de cellules normales (sujets fumeurs, femmes enceintes, etc..)

Elle permettrait de dépister 80 % des cancers,surtout les cancers peu évolués. Ce serait en quelque sorte une protéine spécifique des états débutants : elle correspondrait ainsi parfaitement avec la très bonne sensibilité du procédé objet de la présente invention.

**Revendications**

1. Procédé de détermination de la présence ou de l'absence d'un ligand [X] dans un échantillon fluide à tester, ledit procédé, qui met en oeuvre un dispositif filtrant et une réaction du type antigène/anticorps :

$$X + anti(X) \Rightarrow X\text{-}anti(X) \text{ ou } E^* + anti(E^*) \Rightarrow E^*\text{-}anti(E^*)$$

où E* est une enzyme de marquage, étant caractérisé en ce que

(1°) on met en contact, dans une première zone, l'échantillon fluide à tester susceptible de contenir le ligand [X] avec un anticorps marqué par une enzyme [anti(X)-E*], pour obtenir un complexe de formule :

$$X\text{-}anti(X)\text{-}E^*$$

quand ledit ligand X est présent dans ledit échantillon,

(2°) on met en contact, dans une seconde zone, le complexe X-anti(X)-E* ainsi obtenu quand X est présent dans l'échantillon à tester, ou l'anticorps marqué anti(X)-E* quand X n'est pas présent dans ledit échantillon, avec un anticorps immobilisé spécifique de l'enzyme de marquage de structure:

$$\vdash anti(E^*)$$

où ⊢ symbolise l'immobilisation de l'anticorps anti(enzyme de marquage), ledit anticorps ⊢anti(E*) ne reconnaissant pas l'enzyme de marquage dans le complexe X-anti(X)-E* mais reconnaissant ladite enzyme de marquage dans l'anticorps marqué anti(X)-E* qui est immobilisé, selon les mécanismes :

$$\vdash anti(E^*) + X\text{-}anti(X)\text{-}E^* \Rightarrow \text{pas de réaction}$$

$$\vdash anti(E^*) + anti(X)\text{-}E^* \Rightarrow \vdash anti(E^*)\text{-}E^*\text{-}anti(X)$$

puis
(3°) on révèle, dans une troisième zone, l'enzyme de marquage E* du complexe X-anti(X)-E* au moyen d'un substrat spécifique de ladite enzyme de marquage, pour apprécier la présence dudit ligand X dans l'échantillon à tester.

**2.** Procédé suivant la revendication 1, dans lequel ladite première zone est une membrane poreuse contenant ou supportant l'anticorps marqué par l'enzyme anti(X)-E*.

**3.** Procédé suivant la revendication 1, dans lequel ladite deuxième zone, qui contient l'anticorps immobilisé ⊢anti (E*), est solide et poreuse.

**4.** Procédé suivant la revendication 1, dans lequel ladite troisième zone, qui contient le substrat spécifique de l'enzyme de marquage E*, est poreuse et retient le fluide qui a migré depuis la première zone.

**5.** Procédé suivant la revendication 1, dans lequel ladite première zone comporte plusieurs réactifs anti(X)-E* marqués au moyen de la même enzyme.

**6.** Procédé suivant la revendication 1, dans lequel avant le passage sur ladite première zone, l'échantillon fluide à tester est mis en contact avec un anticorps anti(X) immobilisé pour fixer l'excès de X susceptible d'être présent dans ledit échantillon.

**7.** Procédé suivant la revendication 1, dans lequel X est un antigène et anti(X)-E* est un anticorps Fab ou $F(ab')_2$ couplé à l'enzyme de marquage.

**8.** Dispositif de détermination d'un ligand X dans un échantillon fluide à tester, caractérisé en ce qu'il comprend un système filtrant comportant:

- une première zone poreuse comportant un matériau marqué non immobilisé anti(X)-E*,
- une seconde zone poreuse comportant un matériau immobilisé ⊢anti(E*), qui lors de la migration réagit avec anti(X)-E* mais ne réagit pas avec le complexe X-anti(X)-E* formé dans la première zone quand X est présent dans ledit échantillon à tester, et
- une troisième zone comportant un substrat spécifique de l'enzyme de marquage pour révéler le complexe X-anti(X)-E* quand X est présent dans ledit échantillon à tester.

**Patentansprüche**

**1.** Nachweisverfahren zur Bestimmung der Anwesenheit oder der Abwesenheit eines Liganden [X] in einer zu testenden flüssigen Probe mit Hilfe einer Filtrierungsvorrichtung und einer Antigen-/Antikörperreaktion des folgenden Typs:

$$X + Anti(X) \Rightarrow X\text{-}Anti(X) \text{ oder } E^* + Anti(E^*) \Rightarrow E^*\text{-}Anti(E^*)$$

wobei E* ein Markierungsenzym ist,
dadurch gekennzeichnet, daß

(1) in einer ersten Zone die zu testende Probe, von der vermutet wird, daß sie den Liganden [X] enthält, mit einem Enzym-markierten Antikörper [Anti(X)-E*] in Kontakt gebracht wird, um einen Komplex mit folgender Formel zu bilden:

$$X\text{-}Anti(X)\text{-}E^*$$

falls der Ligand X in der Probe anwesend ist;

(2) in einer zweiten Zone der Komplex X-Anti(X)-E*, der bei Anwesenheit von X in der zu testenden Probe erhalten wurde, oder der Enzym-markierte Antikörper Anti(X)-E*, der erhalten wurde, falls X nicht in der zu testenden Probe war, in Kontakt mit einem immobilisierten Antikörper gebracht wird, der spezifisch für das Markierungsenzym ist und der folgende Struktur hat:

$$\vdash\text{Anti}(E^*),$$

wobei ⊢ die Immobilisierung des Antikörpers Anti(Markierungsenzym) kennzeichnet, der Antikörper⊢Anti(E*) das Markierungsenzym in dem Komplex X-Anti(X)-E* nicht erkennt, aber das Markierungsenzym an dem markierten Antikörper Anti(X)-E* erkennt und bindet - aufgrund der folgenden Reaktionen:

$$\vdash\text{Anti}(E^*) + \text{X-Anti(X)-}E^* \Rightarrow \text{keine Reaktion}$$

$$\vdash\text{Anfi}(E^*) + \text{Anti(X)-}E^* \Rightarrow \vdash\text{Anti}(E^*)\text{-}E^*\text{-Anti(X)}$$

und
(3) in einer dritten Zone das Markierungsenzym E* im Komplex X-Anti(X)-E* mit Hilfe eines spezifischen Substrates des Markierungsenzyms nachgewiesen wird, um die Anwesenheit des Liganden X in der zu testenden Probe nachzuweisen.

**2.** Verfahren nach Anspruch 1, dadurch gekennzeichnet, daß
die erste Zone eine poröse Membran ist, die den Enzym-markierten Antikörper Anti(X)-E* enthält oder trägt.

**3.** Verfahren nach Anspruch 1, dadurch gekennzeichnet, daß
die zweite Zone, die den immobilisierten Antikörper⊢Anti(E*) enthält, fest und durchlässig ist.

**4.** Verfahren nach Anspruch 1, dadurch gekennzeichnet, daß
die dritte Zone, die das spezifische Substrat für das Markierungsenzym E* enthält, porös ist und die Flüssigkeit aufnimmt, die aus der ersten Zone einwandert.

**5.** Verfahren nach Anspruch 1, dadurch gekennzeichnet, daß
die erste Zone mehrere reaktive Anti(X)-E* mit unterschiedlicher Spezifität enthält, die mit demselben Enzym markiert sind.

**6.** Verfahren nach Anspruch 1, dadurch gekennzeichnet,
daß die zu testende flüssige Probe vor dem Eintritt in die erste Zone in Kontakt mit einem immobilisierten Antikörper Anti(X) gebracht wird, um einen Überschuß von X zu binden, das in der Probe vermutet wird.

**7.** Verfahren nach Anspruch 1, dadurch gekennzeichnet,
daß X ein Antigen ist und Anti(X)-E* ein Antikörperfragment Fab oder $F(ab')_2$, das an das Markierungsenzym gekoppelt ist.

**8.** Vorrichtung zur Bestimmung eines Liganden X in einer zu testenden flüssigen Probe, dadurch gekennzeichnet, daß die Vorrichtung ein
Filtrierungssystem enthält, das umfaßt:

- eine erste poröse Zone, die ein markiertes, nicht immobilisiertes Material Anti(X)E* enthält.
- eine zweite poröse Zone, die ein immobilisiertes Material⊢Anti(E*) enthält, das mit Anti(X)-E* während dessen Wanderung reagiert, aber das nicht mit dem Komplex X-Anti(X)-E* reagiert, der in der ersten Zone gebildet wird, wenn X in der zu testenden Probe vorhanden ist, und
- eine dritte Zone, die ein spezifisches Substrat für das Markierungsenzym enthält, um den Komplex X-Anti(X)-E* nachzuweisen, wenn X in der zu testenden Probe vorhanden ist.

## Claims

1. A method for determining the presence or the absence of a ligand [X] in a fluid sample to be tested, said method, which implies the use of a filtering device and a reaction of the antigen/antibody type :

$$X + anti(X) \Rightarrow X\text{-}anti(X) \text{ or } E^* + anti(E^*) \Rightarrow E^*\text{-}anti(E^*),$$

wherein E* is a labelling enzyme, comprising

(1°) bringing into contact, in a first zone, the fluid sample to be tested and susceptible to containing the ligand [X] with an enzyme-tagged antibody [anti(X)-E*], in order to obtain a complex of the formula :

$$X\text{-}anti(X)\text{-}E^*$$

when said ligand X is present in said sample,
(2°) bringing into contact, in a second zone, the complex X-anti(X)-E* thus obtained when X is present in the sample to be tested, or the tagged antibody anti(X)-E* when X is not present in said sample, with an immobilized antibody specific to the labelling enzyme and of the structure

$$\vdash anti(E^*)$$

wherein ⊢ represents the immobilization of the anti(labelling enzyme)antibody, whereby said antibody ⊢anti (E*) wherein ⊢ does not recognise the labelling enzyme in the complex X-anti(X)-E* but does recognise said labelling enzyme in the tagged antibody which is immobilized, according to the mechanisms :

$$\vdash anti(E^*) + X\text{-}anti(X)\text{-}E^* \Rightarrow \text{no reaction}$$

$$\vdash anti(E^*) + anti(X)\text{-}E^* \Rightarrow \vdash anti(E^*)\text{-}E^*\text{-}anti(X)$$

then
(3°) revealing, in a third zone, the labelling enzyme of the complex X-anti(X)-E* with a substrate specific to said labelling enzyme, in order to appreciate the presence of said ligand X in the sample to be tested.

2. A method according to claim 1 wherein said first zone is a porous membrane containing or supporting the tagged antibody anti(X)-E*.

3. A method according to claim 1 wherein said second zone, which contains the immobilized antibody ⊢anti(E*), is solid and porous.

4. A method according to claim 1 wherein said third zone, which contains the substrate specific to the labelling enzyme, is porous and retains the fluid which migrated from the first zone.

5. A method according to claim 1 wherein said first zone comprises several reagents anti(X)-E* which are tagged with the same enzyme.

6. A method according to claim 1 wherein said fluid sample to be tested is brought into contact, before passing on said first zone, with an immobilized antibody anti(X) in order to immobilize the excess of X susceptible to be present in said sample.

7. A method according to claim 1 wherein said ligand X is an antigen and said anti(X)-E* is a Fab or $F(ab')_2$ antibody coupled with the labelling enzyme.

8. A device for determining a ligand X in a fluid sample to be tested, which comprises a filtering system containing

- a first porous zone having a non-immobilized enzyme-tagged antibody anti(X)-E*,
- a second porous zone having an immobilized antibody ⊢anti(E*) which during migration reacts with anti(X)-E* but does not react with the complex X-anti(X)-E* formed in the first zone when X is present in said sample to be tested, and
- a third zone having a substrate specific to the labelling enzyme for revealing the complex X-anti(X)-E* when X is present in said sample to be tested.

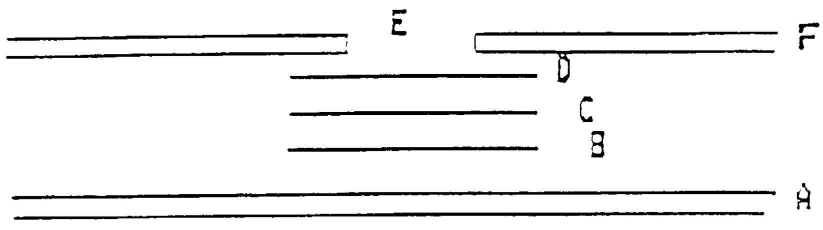


Figure 1

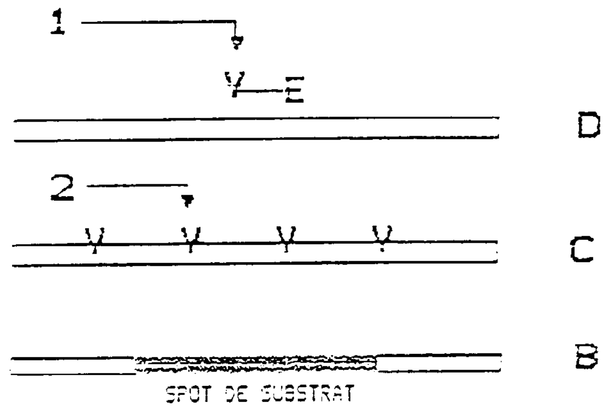


Figure 2

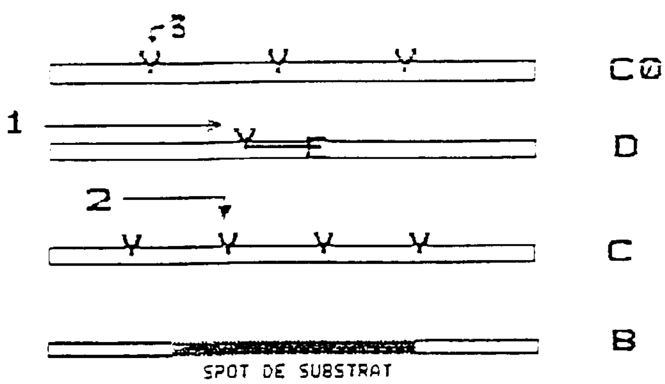


Figure 3

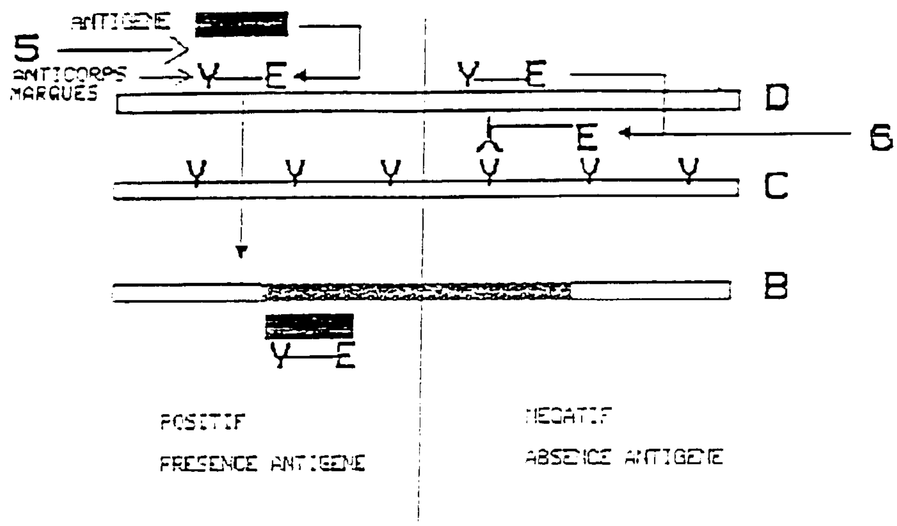


Figure 4

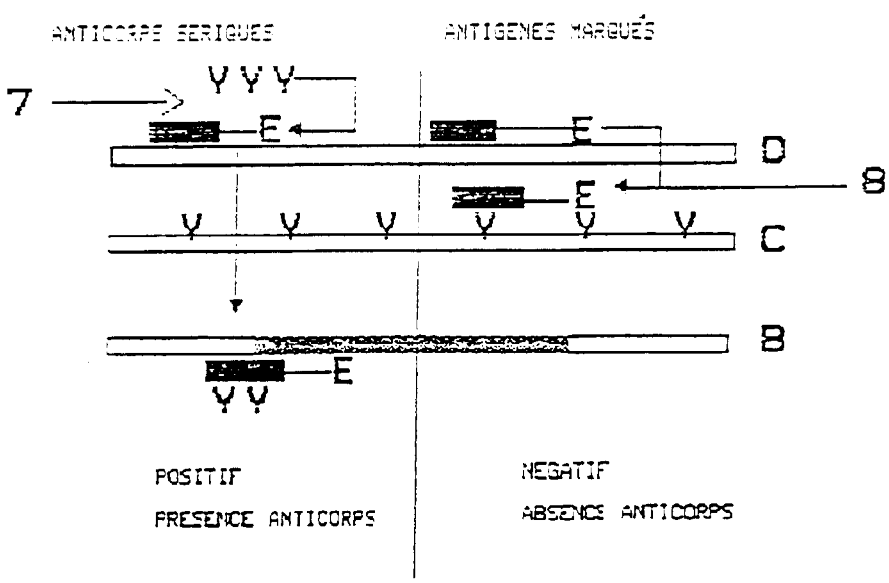


Figure 5

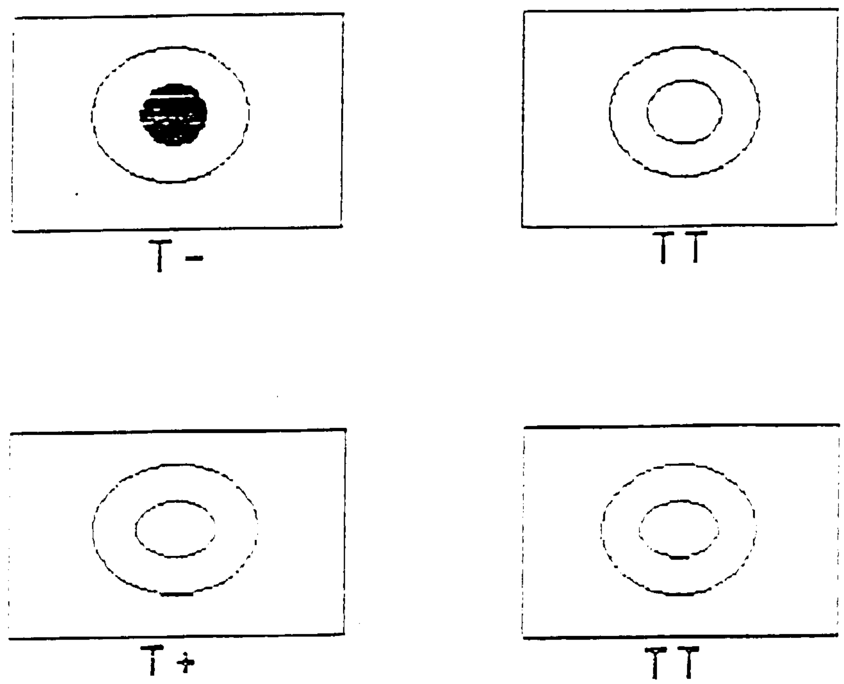


Figure 6

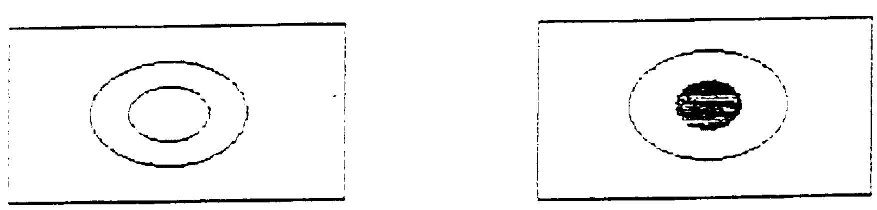

Figure 7

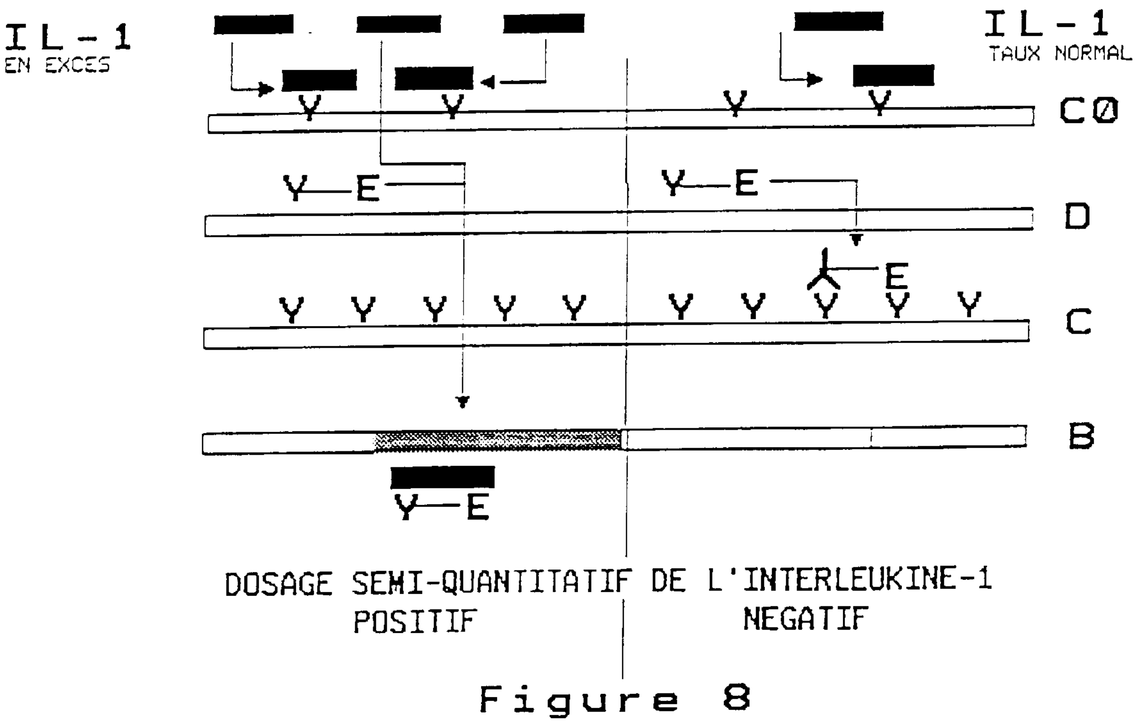
IL-1
EN EXCES
IL-1
TAUX NORMAL
CØ
D
C
B
E
E
E
E
DOSAGE SEMI-QUANTITATIF DE L'INTERLEUKINE-1
POSITIF
NEGATIF

Figure 8

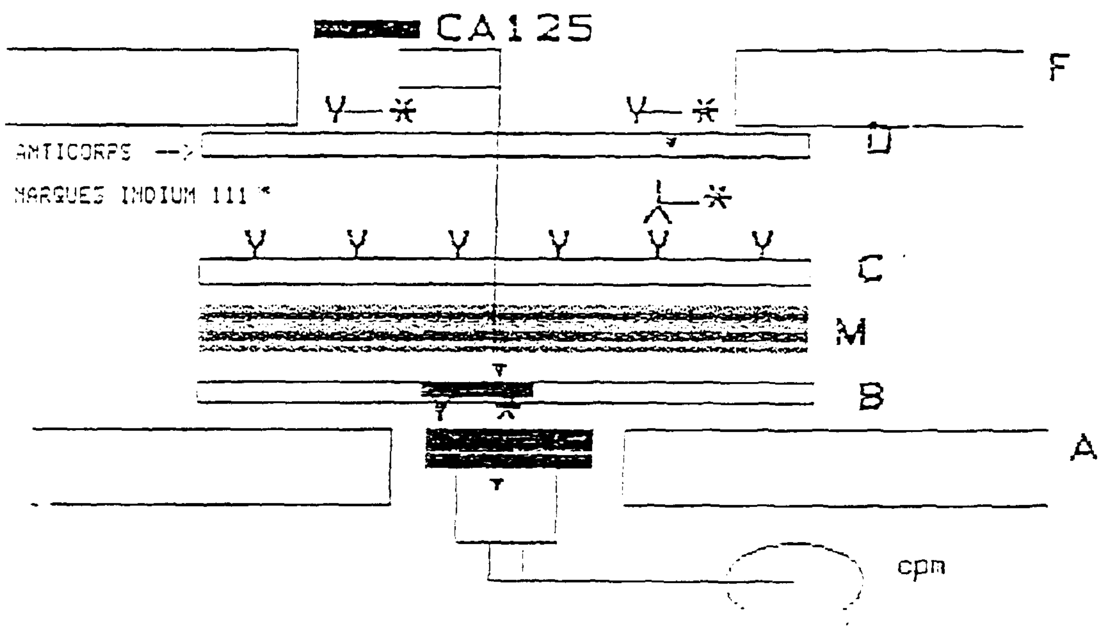


Figure 9

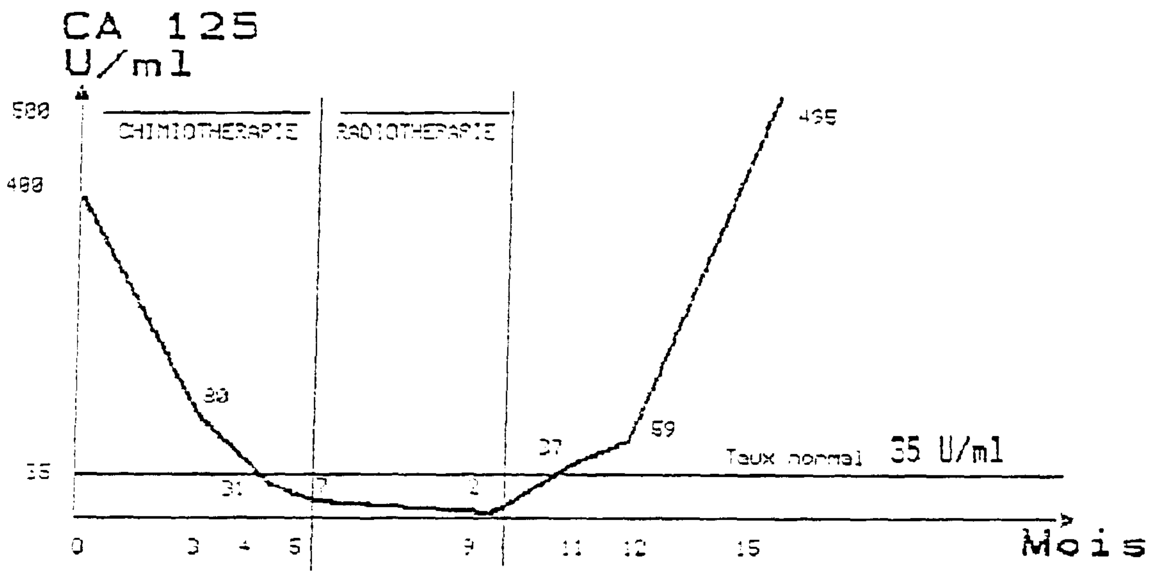
CA 125
U/ml
500
400
CHIMIOTHERAPIE
RADIOTHERAPIE
495
80
59
37
Taux normal 35 U/ml
35
31
7
2
0
3
4
5
9
11
12
15
Mois

Figure 10